# EUROPEAN PATENT SPECIFICATION

(11) **EP 2 328 627 B1**
(45) Date of publication and mention of the grant of the patent: **26.12.2012**
(21) Application number: 09785549.8
(22) Date of filing: 28.08.2009
(51) Int. Cl.: A61L 27/34

(54) **ANTIMICROBIAL COATING**
ANTIMIKROBIELLE BESCHICHTUNG
REVÊTEMENT ANTIMICROBIEN

(30) Priority: 29.08.2008 GB 0815731
(43) Date of publication of application: 08.06.2011
(73) Proprietor: Sheffield Hallam University, South Yorkshire S1 1WB (GB)
(72) Inventor: WANG, Heming, Sheffield, South Yorkshire S1 1WB (GB); AKID, Robert, Sheffield, South Yorkshire S1 1WB (GB); SMITH, Tom, Sheffield, South Yorkshire S1 1WB (GB)
(74) Representative: Stuttard, Garry Philip
(86) International application number: PCT/GB2009/051088
(87) International publication number: WO 2010/023483

(56) References cited:
- DE-A1-102005 013 857
- US-A1- 2005 244 449
- US-A1- 2007 071 789
- NABLO B J ET AL: "Nitric oxide-releasing sol-gels as antibacterial coatings for orthopedic implants" BIOMATERIALS, ELSEVIER SCIENCE PUBLISHERS BV., BARKING, GB LNKD- DOI:10.1016/J.BIOMATERIALS.2004.03.031, vol. 26, no. 8, 1 March 2005 (2005-03-01), pages 917-924, XP025280400 ISSN: 0142-9612 [retrieved on 2005-03-01]
- STOBIE ET AL: "Prevention of Staphylococcus epidermidis biofilm formation using a low-temperature processed silver-doped phenyltriethoxysilane sol-gel coating" BIOMATERIALS, ELSEVIER SCIENCE PUBLISHERS BV., BARKING, GB LNKD- DOI:10.1016/J.BIOMATERIALS.2007.10.057, vol. 29, no. 8, 3 December 2007 (2007-12-03), pages 963-969, XP022398643 ISSN: 0142-9612

## Description

The present invention relates to an antimicrobial sol-gel derived coating and in particular, although not exclusively, to a solid organic-inorganic oxide network chemically bonded to a substrate, the network comprising an antimicrobial releasably captured within the organic- inorganic oxide network.

In most developed countries and as the general population ages, the number of arthroplasty surgical procedures undertaken has increased greatly over the last 20 years. Arthroplasty refers to an operative procedure, in which an arthritic or damaged joint, or joint surface is replaced, or remodelled to restore joint mobility.

The most successful and common form of arthroplasty is the surgical replacement of a dysfunctional joint or joint surface with a prosthetic. A typical example is total hip arthroplasty involving replacement of both the acaetabulum (hip socket) and the head and neck of the femur.

Arthroplasty procedures of the type indicated above may be divided into two general types namely, cemented and cementless procedures. In a cemented procedure a bone cement such as poly(methyl 2-methylpropenoate) is used to attach the prosthetic components to the bone. This provides a strong and rapid bond between the prosthetic and bone. In a cementless procedure, porous materials are utilised to create a prosthetic implant so as to facilitate bone ingrowth and accordingly provide a strong and direct method of biologically fixating the prosthetic.

It has been found that cement based arthroplasty is disadvantageous due to wear of the cement caused by joint manipulation over time. This leads to the formation of stress cracks in the cement and ultimately cement erosion and an unstable joint. Biological fixation, without cement, is therefore advantageous albeit with a slower recovery rate due to the time required for biological fixation (bone in growth into the prosthetic).

A further, more considerable disadvantage with cementless arthroplasty is post operative infections. With cement based procures an antibiotic is typically incorporated within the cement matrix to avoid any such post operative infections which, are a common complication. Serious infection accounts for a modest proportion of post surgical exchange or loss of the prosthetic. The pathogenesis of the prosthetic joint infection results from the formation of a bacterial bio-film resultant from bacteria adhering to the surface of the prosthetic joint.

Treatment of prosthetic joint infections is typically surgery with continued administration of antibiotics over long periods of time. As indicated above, cement based arthroplasty procedures significantly reduce the risk of surgical infection. In particular, gentamicin loaded bone cement has been shown, in isolation, to reduce infection rate by almost a factor of four. The antibiotic loaded bone cement provides a direct, continuous prophylactic dose to the infected area. However, the antibiotic release into the bloodstream must be controlled so as to avoid toxic and allergic responses.

There is therefore a need for a substrate, and in particular an implantable substrate, suitable for use with cementless arthroplasty procedures that exhibits antibacterial characteristics to significantly reduce or preferably eliminate post operative infections.

US2007/0071789 describes implantable medical devices comprising sol-gel coatings for the controlled release of bioactive agents (e.g. antimicrobials). Matrix compositions for the coatings can comprise a sol-gel derived organically modified silane and a hybrid oxide comprising an organically modified silane. Organically modified silanes can be added to the sol-gel mixture in order to modify the properties of the coating. Multi-layer sol-gel coatings are also disclosed.

DE102005013857 discloses an article, such as a medical device, coated with a porous sol-gel layer (preferably SiOx-Sol-Gel), which comprises at least one antimicrobial agent in the form of nanoparticles. Organo-siloxanes can further be included in the layer for the improvement of the sol-gel properties.

Biomaterials 26 (2005) 917-924 (NABLO B. J. et al) describes (NO)-releasing sol-gels as antibacterial coatings for orthopedic devices. Medical-grade stainless steel is coated with a sol-gel film of 40% N-aminohexyl-N-aminopropyltrimethoxysilane and 60% isobutyltrimethoxysilane and the diamine groups in these films are converted to diazeniumdiolate NO donors.

Biomaterials 29 (2008) 963-969 (STOBIE et al) describes silver-doped phenyltriethoxysilane sol-gel coatings as biofilm inhibitors for medical devices (e.g. catheters, implants).

WO 2006/115805 and Biomaterials 28 (2007) 1721-1729 (RADIN et al) disclose a biocompatible composite for use in contact with body fluids for orthopaedic implantation. The composites are formed as a sol-gel coating layered onto a substrate with a pharmaceutically active compound incorporated in the coating. In particular, the sol-gel layer may comprise an antibiotic that is configured to be released following implantation of the composite so as to reduce the risk of post surgical infection.

However, a number of disadvantages exist with conventional antibacterial sol-gel coatings including primarily, the integrity of such coatings and the rate and duration of antibiotic release *in vivo.*

Accordingly, the inventors provide a substrate comprising a sol-gel derived coating that is configured for the controlled release of an antimicrobial. In particular, the coating is configured such that the antimicrobial is captured within an organic-inorganic oxide network and is released only at the appropriate time (during and post surgery) allowing the substrate to be stored prior to use, without losing its antimicrobial functionality. As indicated above, controlled release is of considerable importance given the risk of toxic and allergic responses to the antimicrobial.

The present sol-gel derived coating is configured specifically to encapsulate the antimicrobial and to release it only in response to the introduction of a fluid, in particular a biological fluid, to and from the organic-inorganic oxide network.

The inventors provide both a method of preparing the antimicrobial coating and an antimicrobial coated substrate. Turning firstly to the method, the formulation of the sol prior to application on the substrate, has been optimised to both increase the available storage time of the sol prior to application and to ensure the effectiveness of the antimicrobial-substrate coating is not reduced, at least below required levels. Accordingly, there may be provided a two stage preparatory method in which the antimicrobial is independently suspended in a solution optimised for both storage of the antimicrobial and to avoid unwanted reactions with the sol that is to create the porous network at the substrate surface. The separately prepared sol may then be mixed with the antimicrobial preparation a short time prior to coating.

The organic-inorganic oxide sol is optimised such that the resultant solid organic-inorganic oxide network does not impede a continuous or otherwise controlled release of the antimicrobial during and after surgical implantation (where the substrate is a prosthetic, for example). Importantly, the antimicrobial is immobilised within the dry, cured coating allowing the implant to be physically handled and manipulated by a surgeon during arthroplasty procedures.

However, when a fluid, in particular a bodily fluid, is introduced into the porous network, the antimicrobial is mobilised to provide controlled release into the patient. As will be appreciated, the encapsulation and indeed the dispersion of the antimicrobial within the sol-suspension mixture prior to coating is important to optimise so as to achieve the desired concentration distribution of antimicrobial through the thickness of the coating. Moreover, the curing conditions are also important to ensure the antimicrobial is capable of being mobilised by introduction of the fluid.

The inventors provide a biocompatible coating that is optimised to allow the creation of a thick coating that adheres firmly to the substrate. The coating comprises enhanced durability over known systems advantageously exhibiting wear resistance whilst providing a controlled, sustained release of the biologically active compound from the porous network.

The inventors have realised that by forming the coating via a sol-gel process using a polysiloxane and optionally further precursors such as a silane and/or a silicate for the sol component, a porous network is created that is ideally suited as a coating for biological implants such as prostheses and fixation devices, including screws, pins, bone plugs and the like. Utilising a polysiloxane precursor, in contrast to a siloxane monomer is advantageous for a number of reasons. In particular, the polysiloxane precursor provides control of coating thickness; increased bonding strength to the substrate; improved flexibility of the coating; a controlled porosity of the network; tailoring of curing temperature and hydrophobicity; and a crack free, non-brittle coating.

The polysiloxane effectively reduces the extent of the condensation reaction and accordingly loss of solvent during the sol-gel process. This provides enhanced coating flexibility and a crack free, non-brittle structure. The coating thickness is controlled as the polysiloxane precursor may be readily crossed linked with other nano particles forming part of the resultant network. Cross linking agents and curing agents may be incorporated at the sol-gel stage so as to facilitate network formation during gelation.

The long-chain polysiloxane, being substantially linear, enables easy control of the porosity. Utilising linear polysiloxane also facilitates cross linking between the Si-O backbone.

In particular, the hydrophobicity has been found to be particularly important for the controlled release of the antimicrobial. The hydrophobicity may be tailored by variation of any one or a combination of i) the concentration of polysiloxane in the sol-gel (and the resulting coating network); ii) the chain length of the polysiloxane; and iii) the extent and nature of functional groups extending from the polysiloxane.

The polysiloxane may comprise function groups/or functionalised side chains extending from the main Si-O backbone. These functional groups and side chains may comprise any oxygen or nitrogen based groups with functionalised side chains comprising for example, acrylic, epoxy or other functionalised groups including organosilanes and/or hybrid organic-inorganic silicate, siloxane and silane compounds.

The synergistic combination of the Si-O and Si-C bonds provides for the possibility of creating thick porous coatings of the order of 5 -10 µm. This is in contrast to conventional sol-gel films in this field where maximum coating thickness of not greater than 200 nm are possible without undesirable cracking. Importantly, coating may be configured to remain in contact with the substrate for significant time periods (of the order of months or years) prior to degradation. Accordingly, the pharmaceutically active compound, incorporated within the porous network, may be released into a biological environment for a much greater time period over existing systems. The longevity of the coating is also advantageous so as to prevent corrosion and degradation of the implant after the pharmaceutically active compound has been completely released from the network.

The present coating may be formed as a single or multiple layer system on the substrate. Importantly, the antimicrobial is released from the network whilst the coating is maintained at the substrate. This is in contrast to that proposed by Radin systems in which the releases mechanism is by way of loss and degradation of outermost regions of the coating. Such coatings are disclosed as dissolving completely over a period of only two or three days.

By repeating coating and curing steps during formation, it is possible to create a multilayer sol-gel derived coating in which, for example, a layer positioned towards the substrate-coating interface comprises different chemical and/or physical/mechanical properties to a layer at the outermost region of the coating. Importantly, the hydrophobic property of the coating may be tailored by adjustment of the relative concentrations of the silane, silicate and/or polysiloxane precursors so as to optimise the release rate of the antimicrobial from the network and the stability of the coating at the substrate. Significant reductions in curing times are also possible with the present invention. In particular, at room temperature curing times are less than 50% of the prior art systems so as to achieve the desired crosslinking/condensation of the network. Cure times of around 1 hour may be achieved at curing temperatures of between 55° - 75°.

According to a first aspect of the present invention there is provided a substrate having a: hybrid coating chemically bonded to the substrate, the coating obtainable by a sol-gel process using a polysiloxane ; the coating comprising a polysiloxane based network of silicon-carbon bonds and silicon-oxygen bonds; and an antimicrobial component releasably captured within the network and capable of defusing from the network in response to a fluid introduced into the coating.

Preferably, the network is a porous network allowing fluid to flow into and out of the network. Porosity of the network may be controlled at the sol-gel stage of the process so as to achieve the desired release rate of antimicrobial.

Optionally, the substrate comprises a metal, in particular stainless steel, a titanium based alloy, a cobalt based alloy and/or a chromium based alloy and/or a magnesium based alloy. Alternatively the substrate may comprise a ceramic, a plastic or mineral based material. The substrate may comprise a medical tool or medical apparatus associated with patient care and surgical procedures. In addition, the substrate may comprise a structure associated with food preparation including by way of example, food preparation surfaces. Moreover, the substrate may comprise structures designed to be frequently contacted by liquid, in particular water, such as wash basins, toilets, baths, showers and tiles etc.

Optionally, the coating may comprise a dopant species captured or chemically bonded at the hybrid organic-inorganic oxide network. In particular, the present coating is preferably formed by incorporating nano particles at the sol-gel stage of coating formation. The nano particles may comprise a silane, a silicate and/or other dopant particles such as γ-Al₂O₃ and hydroxyapatite. The linear polysiloxanes, within the network are preferably chemically bonded to one another by cross linking agents. The cross linking agents may comprise non-functionalised organic hydrocarbons or functionalised hydrocarbons or other organic, inorganic and/or organic-inorganic cross linking agents. The nano particles incorporated in the sol-gel phase chemically bond to the polysiloxane during the condensation process. The resulting network comprises substantially linear polysiloxane with Si-O repeating units and organic side chains extending from the main Si-O backbone. The organic side chains may comprise any alkyl, aryl and/or mixed alkyl-aryl groups. These alkyl or aryl groups may be substituted with additional functionalised groups along the Si-O backbone, where the functionalised groups comprises any elements selected from period table groups 5 to 7 including in particular nitrogen, phosphorus, oxygen, sulphur and chlorine.

Where the organic side group, directly bonded to the Si-O backbone is alkyl, the alkyl group may comprise between 1 to 20 carbon atoms. Optionally, the alkyl, aryl and/or mixed alkyl-aryl groups that are attached directly to the Si-O backbone may be functionalised by comprising nitrogen, phosphorous, oxygen, sulphur and/or chlorine atoms. Optionally, the Si-O backbone may comprise at least one functional side chain bonded directly to either the Si-O backbone or at least one of the alkyl, aryl or alkyl-aryl side groups.

According to the preferred implementation, the polysiloxane is substantially linear. Alternatively, the polysiloxane may be branched at more than one regions along the length of the main Si-O backbone.

The antimicrobial may comprise a uniform concentration distribution through the coating thickness from an external facing region to the substrate-coating interface. Alternatively, the coating may comprise a substantially non-uniform concentration distribution of the antimicrobial through the coating thickness from the external facing region to the substrate-coating interface.

The coating may comprise a multilayer structure, each layer having a different concentration of the antimicrobial. This would allow different concentrations of antimicrobial to be released over time. For example, a concentration rich layer of antimicrobial may be provided towards an outermost region of the coating so as to release large concentrations during and immediately after surgery whilst an inner coating layer may have a relatively lower antimicrobial concentration. The multilayer structure may be formed by multiple sol-gel coating and curing steps.

Optionally, the substrate may comprise a liquid, the liquid being contained within the inorganic network such that the antimicrobial is dissolved and mobilised within this liquid phase. Additional means may be provided so as to seal the liquid within the organic-inorganic network so as to prevent liquid loss through evaporation or the like, during storage of the substrate.

Optionally, for multi-layer systems a region of the coating positioned towards the substrate-coating interface may be more hydrophobic than a region positioned towards the outermost surface of the coating. Also, the coating at the substrate-coating interface may comprise a greater hardness than the outmost region of the coating. Optionally, the coating or outermost layer of the coating at the substrate-coating interface may comprise hydoxyapatite so as to improve compatibility.

According to a second aspect of the invention there is provided a biocompatible implant for a human or animal comprising the coating obtainable by a sol-gel process using a polysiloxane as described herein. In particular, the implant may be a prosthetic or fixation device, including by way of example, bone screws, pins, rivets or plugs.

Optionally, the coating may comprise nano particles incorporated within the network during the sol-gel process. These nano particles may comprise TiO₂, γ-Al₂O₃ and in particular nano-hydoxyapatite.

The present coating may also be functionalised by the addition of one or more components configured to promote bone regrowth at the region of the coating, where for example the coating is applied to a prosthesis. Such functionalised components may also be configured to prevent destruction of the bone by osteoclasts around the prosthesis, to generally promote desired cell proliferation and to improve the performance of the prosthesis by molecular interaction and/or chemical reaction with the host's biological system in vivo. Such additional biological functionalised components may include: osteogenic proteins (including but not restricted to one or more recombinant human bone morphogenic proteins); carboxymethyl chitosan; other biologically active proteins, DNA, extracellular matrix components and analogues thereof; other molecules; multi-molecular complexes and assemblies, and nanoparticles.

Dopant nanoparticles considered to be advantageous for bone regrowth include active species containing calcium and/or phosphorous and agents derived from vitamins.

According to a third aspect of the present invention there is provided a method of preparing a substrate, the method comprising: preparing a sol using a polysiloxane ; making a preparation comprising an antimicrobial; mixing the sol and the antimicrobial component together to form a mixture; coating the substrate with the mixture; curing the mixture on the substrate to form a sol-gel derived hybrid organic-inorganic coating chemically bonded to the substrate, the coating comprising a polysiloxane based network of silicon-carbon bonds and silicon-oxygen bonds; wherein the antimicrobial component is releasably captured within the network and capable of defusing from the network in response to a fluid introduced onto the coating.

Preferably, the preparation further comprises sterilising the coating, for example prior to implantation. Sterilisation may comprise exposure of the coating to gamma radiation, or by any other suitable physical and/or chemical means.

The present coating may utilise any silicate based precursor including specifically an organosilicate and/or a silane based precursor including in particular an organosilane. Further, the polysiloxane component may comprise a form of polysiloxane including in particular an organopolysiloxane.

The term 'hybrid coating' within the specification refers to a sol-gel derived coating formed from at least two different silicon based precursors. Accordingly the hybrid coating of the subject invention comprises at least a first silicon centre, derived from a first precursor bonded to carbon and a second silicon centre, derived from a second precursor bonded to oxygen. That is, at least two silicon centres differ throughout the network by the number of respective carbon and/or oxygen bonds at each silicon centre.

In particular, the sol-gel derived polysiloxane based coating may be derived from any one or a combination of the following additional precursors incorporated within the coating network during the sol-gel phase: any organically modified silane selected from the group consisting of alkylsilanes; methyltrimethoxysilane; methyltriethoxysilane; dimethyldiethoxysilane; trimethylethoxysilane; vinyltrimethoxysilane; vinyltriethoxysilane; ethyltriethoxysilane; isopropyltriethoxysilane; butyltriethoxysilane; octyltriethoxysilane; dodecyltriethoxysilane; octadecyltriethoxysilane; aryl-functional silanes; phenyltriethoxysilane; aminosilanes; aminopropyltriethoxysilane; aminophenyltrimethoxysilane; aminopropyltrimethoxysilane; acrylate functional silanes; methacrylate-functional silanes; acryloxypropyltrimethoxysilane; carboxylate; phosphonate; ester; sulfonate; isocyanate; epoxy functional silanes; chlorosilanes; chlorotrimethylsilane; chlorotriethylsilane; chlorotrihexylsilane; dichlorodimethylsilane; trichloromethylsilane; N,O-Bis (trimethylsilyl)-acetamide (BSA); N,O-Bis (trimethylsilyl) trifluoroacetamide (BSTFA); hexamethyldisilazane (HMDS); N-methyltrimethylsilyltrifluoroacetamide (MSTFA); N-methyl-N-(t-butyldimethylsilyl)trifluoroacetamide (MTBSTFA); trimethylchlorosilane (TMCS); trimethylsilyimidazole (TMSI); and combinations thereof.

The polysiloxane may comprise any one or a combination of the following groups: an alkyl; a substituted alkyl, a halosubstituted, an alkenyl, an alkynyl, a halosubstituted alkynyl, a phenyl, a substituted phenyl, a hydroxylic compound. In particular, the polysiloxane may comprise an organofunctionalised group including in particular a hydroxyl, epoxy alkoxy, silanol, amino or isocyanate group. The polysiloxane may comprise a single repeat unit or may be formed as a two, three, four or five component polysiloxane having different respective repeater units forming part of the Si-O part of the backbone. Specifically, and my way of example, the polysiloxane may comprise any one or a combination of the following compounds: Poly[dimethylsiloxane-co-[3-(2-(2-hydroxyethoxy)ethoxy)propyl]methylsiloxane]; Poly(dimethylsiloxane), bis(3-aminopropyl) terminated; Poly(dimethylsiloxane), diglycidyl ether terminated; Poly(dimethylsiloxane)-graft-polyacrylates; Poly[dimethylsiloxane-co-methyl(3-hydroxypropyl)siloxane]-graft-tetrakis(1,2-butylene glycol); Poly[dimethylsiloxane-co-(2-(3,4-epoxycyclohexyl)ethyl)methylsiloxane]; Poly[dimethylsiloxane-co-(3-aminopropyl)methylsiloxane; Poly[dimethylsiloxane-co-methyl(stearoyloxyalkyl)siloxane] and/or Poly[dimethylsiloxane-co-[3-(2-(2-hydroxyethoxy)ethoxy)propyl]methylsiloxane].

The polysiloxane preferably comprises a minimum repeat number of ten and may comprise ten, a hundred, a thousand or tens of thousands of repeat units within a single polymer backbone.

The term `alkyl' refers to a linear, branched, cyclic, or any combination thereof hydrocarbon. The term 'substituted alkyl' refers to one or more of the hydrogens on the alkyl group being replaced by another substituent, such as cyano, alkyl, nitro, mercapto, alkylthio, halo, alkylamino, dialkylamino, alkoxy, and tri alkoxysilyl. The term 'Substituted phenyl' refers to one or more of the hydrogens on the aromatic ring being replaced by another substituent, such as cyano, alkyl, nitro, mercapto, alkylthio, halo, alkylamino, dialkylamino, and alkoxy.

Optionally, the present coating may be derived from any one or a combination of the following precursors: tetraethoxy orthosilicate (TEOS); methyltriethoxy orthosilicate (MTEOS); phenyltriethoxy orthosilicate (PTEOS); octyltriethoxy orthosilicate (OTEOS); dimethyldiethoxy orthosilicate (DMDEOS); methyltrimethoxy orthosilicate (MTMOS); phenyltrimethoxy orthosilicate (PTMOS); tetramethoxy orthosilicate (TMOS).

The antibacterial component of the present invention may comprise any one or a combination of the following: a living cell, including but not restricted to a bacterial cell. (Optionally where the bacterial cell is immobilised in the form of an endospore); a cell that is no longer living but provides a modified functionality/property to the coating; a component that inhibits or influences the formations ofbiofilms and/or biofouling, including but not restricted to a protease, an inhibitor of quorum sensing or a microorganism that produces either or both of these, where the quorum sensing inhibitor includes but is not restricted to a furanone.

In particular, the antimicrobial component may comprise: aminoglycosides including but not restricted to: gentamicin; amikacin; arbekacin; kanamycin; neomycin; netilmicin; paromomycin; rhodostreptomycin; streptomycin; tobramycin; and/or apramycin.

Alternatively the antibacterial component may comprise: beta-lactams, including in particular cephalosporins, including but not restricted to: Cefazolin; Cefotaxime; Cefoxitin; Ceftazidime; Cefuroxime; Cephalosporin C; and/or Carbapenems inc; and carbapenems, including but not restricted to: dorapenem; meropenem; Imipenem; and/or Ertapenem.

Alternatively the antibacterial component may comprise: alternative beta lactams: Ticarcillin; Clavulanic acid; Co-amoxyclav; Piperacillin/tazobactam; Ampicllin; Benzyl penicillin; Amoxicillin; Oxacillin; Cloxacillin; Flucloxcillin; and/or aztreonam.

In particular, the antimicrobial component may comprise: antimicrobial peptides including but not restricted to: Polymyxin; Nisin.

In particular, the antimicrobial component may comprise: lipopeptides including but not restricted to: Daptomycin.

In particular, the antimicrobial component may comprise: Glycopeptides including but not restricted to: Vancomycin; teicoplanin.

In particular, the antimicrobial component may comprise: Macrolides including but not restricted to: erythromycin; clarithromycin; azithromycin; Dirithromycin; Roxithromycin; Telithromycin.

In particular, the antimicrobial component may comprise: Lincosamides including but not restricted to Clindamycin.

In particular, the antimicrobial component may comprise: Ketolides including but not restricted to Telithromycin.

In particular, the antimicrobial component may comprise: Tetracyclines including but not restricted to: Tetracycline; Doxycyline; tigecycline.

In particular, the antimicrobial component may comprise: Quinolones including but not restricted to: Nalidixic acid; Ciprofloxacin; Levofloxacin; Gatifloxacin; moxifloxacin; Chloroquin.

In particular, the antimicrobial component may comprise: other antibiotics and antifungal agents including but not restricted to: Rifampicin; Metronidazole; Fusidic acid; Colistin; Fosfomycin; Fluconazole; Caspofungin.

The present coating may be suitable as an antifouling and/or anticorrosion coating. In particular, the present coating may find application as a marine antifouling/anticorrosion coating. The present coating may be suitable as a coating for metals, plastics, glasses, ceramics and/or other metallic, organic and/or inorganic substrates.

A specific implementation of the present invention will now be described, by way of example only and with reference to the accompanying drawings in which:

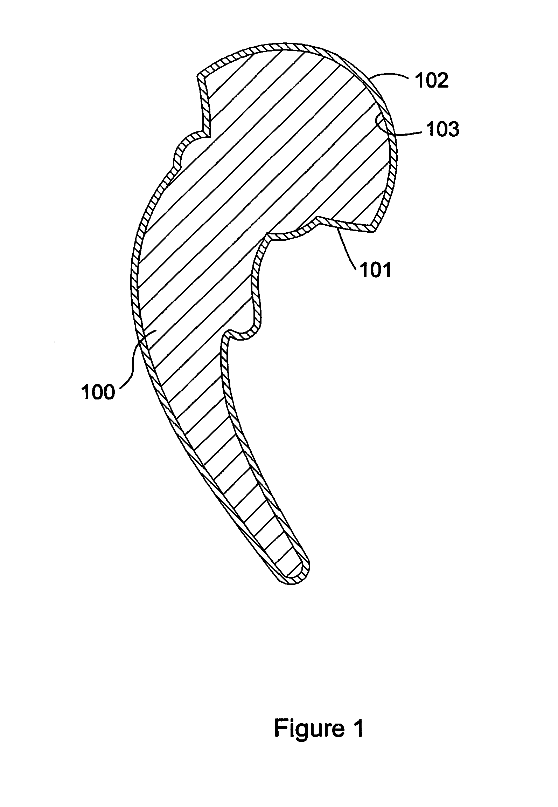

Figure 1 illustrates a cross sectional view of a prosthetic comprising a coating of the subject invention.

The present invention finds particular application within the medical field and in particular for coating devices to be implanted in the human or animal body. Referring to Figure 1, a hip prosthetic 100 is coated with the present antimicrobial encapsulating network 101. Figure 1 illustrates prosthetic 100 coated over its entire surface area with coating 101. As will be appreciated, the coating may be applied over specific regions of the outer surface of the substrate 100 as desired.

The silane and silicate based sol, that forms the bulk of coating 101 is prepared independently of the aqueous-based antimicrobial suspension. This allows the sol-gel component to be optimised to create the desired porous network whilst optimising the antimicrobial suspension to ensure the biologically active species maintains its viability in successfully inhibiting bacterial colonisation.

The silane and silicate based sol and the microbial suspension are then mixed prior to coating on substrate 100. Coating 101 is applied by any conventional technique including dip, spray or spin techniques. Coating 101 is then cured so as to allow the sol-gel to bond chemically with the outmost surface of substrate 100 so as to provide a secure coating resistant to the various torsional, sheer an impact loading forces imparted to prosthetic 100 as the joint is manipulated throughout the lifetime of the implantation. The hybrid organic-inorganic coating can be configured specifically to chemically bind to a plurality of different substrate materials including for example, glass, metal, ceramic and mineral surfaces.

Coating 101 is positioned in direct contact with the outer surface of substrate 100 to form a substrate-coating interface 103. An external facing surface 102 of coating 101 is therefore presented and configured to be in direct contact with either bone or soft biological tissue. The antimicrobial, freely suspended in the sol-suspension mixture, is encapsulated in the solid network of the coating extending between substrate-coating interface 103 to the external facing surface 102 of coating 101.

Multiple coating layers 101 may be applied one on top of another over substrate 100 to form a multilayer structure. This may involve repeated application of the sol-suspension mixture followed by sequential curing of each wet layer. Accordingly, it is possible to produce a multilayered coating having a variable or uniform antimicrobial concentration gradient from outermost surface 102 to substrate-coating interface 103.

An experimental investigation was undertaken utilising the antimicrobial gentamicin encapsulated within a sol-gel coating for potential use as an antibacterial coating/film on a cementless prosthetic.

One aim of the experimental investigation was to determine if gentamicin is released from the coating whilst maintaining its viability as an antibiotic.

A secondary aim was to determine if the sol-gel derived coat could be cured at different temperatures, and in particular a high temperature, without destroying antimicrobial viability. Typically, a high curing temperature provides a stronger chemical bond between coating 101 and substrate 100 and is therefore advantageous.

### Methods and Material

### Example 1

The base hybrid sol was prepared firstly by mixing tetraethoxysilane (TEOS), tetramethylorthosilicate (TMOS), methyltrimethoxysilane (MTMS), and poly(dimethylsiloxane) (PDMS) in ethanol according to the volume ratio of 1:1:1:0.5.2.4. Deionised water was added drop-wise into the base sol-gel sol. Glacial acetic acid or nitric acid was also added as the catalyst to promote hydrolysis and condensation reactions. The pH value of the prepared sol was adjusted to a suitable value in the range 1 to 7.

### Example 2

The base hybrid sol was prepared firstly by mixing tetraethoxysilane, tetramethoylorthosilicate, methyltrimethoxysilane, and Poly[dimethylsiloxane-co-[3-(2-(2-hydroxyethoxy)ethoxy)propyl]methylsiloxane] in ethanol according to the volume ratio 1:1:1:0.2:2.0. Deionised water was added drop-wise into the base sol-gel sol. Glacial acetic acid or nitric acid was added as the catalyst to promote hydrolysis and condensation reactions. The pH value of the prepared sol was adjusted to a suitable value in the range of 1 to 7.

### Substrate Preparation and Antibacterial Agent

Three bacteria were investigated on a glass substrate: Escherichia coli (W3110, k 12 mutant), *Staphylococcus aureus* and a coagulase negative *Staphylococcus epidermis.* The *E. coli* and *S*. *aureus* being laboratory strains and the *S*. *epidermis* had been swabbed and sub cultured from a patient 48hrs prior to the experiment. All methods used were done to stranded aseptic techniques, and all solutions, agars and equipment where sterilized by an autoclave or wiped with an alcohol wipe.

Each of the bacterium where grown on a nutrient agar plate and an agar/gentamicin plate at a concentration of 50 µg per ml. This ensured the bacterial strains used where not resistant to gentamicin. One colony from the bacteria agar plate was used to inoculate a sterilized L-broth of 1% tryptone, 0.5% yeast extract, 0.5% NaCl, 0.2% glucose and left over night in a shaking incubator at 37°C.

The substrate was a standard glass microscope slide sterilized in an autoclave. Three different coatings were tested i) a pure sol-gel coating was used for a control, ii) a 5 mg/ml gentamicin solution mixed with sol-gel to form a 50 µg/ml coating (50ppm) and a iii) 12.5 mg/ml concentration of gentamicin was made from gentamicin sulphate dissolved directly in the sol-gel to provide a 1.25% gentamicin sol-gel coating. Each coating was applied over the microscope slide using 200µl of coating per plate, covered in one single coat and left for 24hrs at room temperature to allow the coat to cure. Four plates were dried at 80°C for 1hr achieving a harder coat to determine if the gentamicin coat was still active after high temperature treatment.

To infect the plates with bacteria, a "sloppy agar" was made up from 0.7g of nutrient agar dissolved in 100ml of Phosphate buffer saline (PBS) and autoclaved forming a jelly like consistency. After melting, 5ml of a inoculate L- broth was pipetted in to 100ml of sloppy agar and mixed with 500µl of this inoculated agar to be pipetted on to a coated plate forming a drop sitting on top of the coat. The plate was then laid flat into a Petri dish which was then placed in to a sealed, airtight, container with damp tissue paper to keep the sloppy agar in a moist environment to prevent drying out. The sealed container was incubated for a set time at 37°C. Each bacterium was placed on a separate coated plate, one plate for each time point of 24hr, 48hr, 96hr and 168hr allowing a review of the relative release rate of the gentamicin and the speed of inhibition and bacterial growth.

After the allotted time the Petri dish was removed from the container and, using aseptic techniques, 0.1g cube was cut from the sloppy agar in the middle of the plate down to the coated layer. This was mixed with 900µl of Ringer's solution in an epidorf tube to produce the first dilution x10⁻¹. Subsequently, 100µl of this dilution was added to the next epidorf tube containing 900µl of Ringer's solution and so on making dilutions though to x 10⁻⁶. Two 20µl drops of each dilution were added to a sectioned nutrient agar plate. When each dilution was plated, the petri dish was incubated for 24hrs to allow viable bacteria to grow producing countable colonies.

The backlight testing kit was an assay of two coloured fluorescent chemicals. The first was SYTO 9 (component A), and the second was propidium iodide (component B). In this experiment a spectrophotometer was used to determine the relative number of living and dead bacteria on the 168hrs plates. The x 10⁻¹ dilution was centrifuged for 15 minutes to produce a pellet of bacteria. The pellet was re-suspended using 1ml of PBS and 3µl component A and B was added and mixed by a vortex. Four readings where taken for each sample, two for each wavelength, one zeroed with PBS the other zeroed with PBS added with 3µl of each component.

### Results

All strains of bacteria were shown to be susceptible to gentamicin at concentrations of 50µg per ml. Viability counts were taken for each bacterium at each time point for each coated sample. Each dilution had two 20µl drops where bacterial colonies were counted with an average taken of the two. If the total counted colonies were between 20-150, this was selected as, counts fewer than 20 are unreliable and over 100 are difficult to distinguish between colonies. This average was divided by the dilution factor which gave the number of colony forming units (CFU) per 20µl. This value was multiplied by 50 to give the CFU per ml for each dilution. The stranded deviation (SD) was determined for each pair of 20µl drops for the SD per CFU per ml.

**Table 1 Colony forming units (CFU) per ml and the corresponding stranded deviation (SD)**

| | **24hr CFU per ml x10⁻³** | | **48hr CFU per ml x10⁻³** | | **96hr CFU per ml x10⁻³** | | **168hr CFU per ml x10⁻³** | |
|---|---|---|---|---|---|---|---|---|
| **S. aurous** | **24 hr** | **24hr SD** | **48 hr** | **48hrs SD** | **96 hr** | **96hr SD** | **168 hr** | **168hr SD** |
| **Control** | 8000 | 88 | 2775 | 724 | 1875 | 123 | 140 | 12 |
| **50ppm** | 3537 | 203 | 2450 | 530 | 0 | 0 | 22 | 17 |
| **1.25%** | 0 | 0 | 0 | 0. | 40497 | 15517 | 0 | 0 |

| | **24hr CFU per ml x10⁻³** | | **48hr CFU per ml x10⁻³** | | **96hr CFU per ml x10⁻³** | | **168hr CFU per ml x10⁻³** | |
|---|---|---|---|---|---|---|---|---|
| **E.coli** | **24 hr** | **24hr SD** | **48 hr** | **48hrs SD** | **96 hr** | **96hr SD** | **168 hr** | **168hr SD** |
| **Control** | 80000 | 3535 | 64500 | 10429 | 14000 | 0 | 3450 | 494 |
| **50ppm** | 4687 | 645 | 7750 | 883 | 0 | 0 | 0 | 0 |
| **1.250** | 0 | 0 | 0 | 0 | 29686 | 890 | 0 | 0 |

| | **24hr CFU per ml x10⁻³** | | **48hr CFU per ml x10⁻³** | | **96hr CFU per ml x10⁻³** | | **168hr CFU per ml x10⁻³** | |
|---|---|---|---|---|---|---|---|---|
| **S. epidermis** | **24 hr** | **24hr SD** | **48 hr** | **48hrs SD** | **96 hr** | **96hr SD** | **168 hr** | **168hr SD** |
| **Control** | 172500 | 15909 | 55125 | 8573 | 52000 | 5303 | 22750 | 3712 |
| **50ppm** | 30250 | 503 | 9250 | 1237 | 37050 | 901 | 0 | 0 |
| **1.250** | 0 | 0 | 0 | 0 | 30033 | 50 | 0 | 0 |

The viable counts for the control coating show a decrease in CFUs over time with the final 168hr control counts been less than 2.2% of the original 24hr control. This is due to the low amount of available nutrients in the sloppy agar, high concentration of bacteria and the long incubation periods resulting in a rapid progression to death phase on the bacteria growth curve. The 50ppm coat showed an immediate reduction in CFUs after 24 hr, when compared to the control, the results being *S*. *aureus* 56%, *E.coli* 95% and *S. epidermis* 83%. The S. epidermis formed a much smaller colony but was much greater (approximately by a factor of 10), in number and its viable count results reflected as much. The 48hrs results are also lower in CFUs for the 50ppm than the control, but E. coli increases in number from the 24 hr.

The high temperature dried coating results indicate the same pattern as the room temperature control and 50ppm coating. Both the high temp control and 50ppm showed a drop in CFU from 24 to 48 hrs, with the 50ppm coat showing a 70.5% reduction in CFU in the first 24hrs which is similar to the room temp sample reductions.

The backlight kit results are relative to each other in terms of how much bacteria was in each sample. The 1.25% sample has the lowest value for living bacteria making it the base line. The control has more live and dead bacteria then the gentamicin coated samples for *S*. *aureus* and *E*. *coli,* which correlate well with the viable counts, that show plenty of growth in the first 24hrs followed by death over time until 168hrs where the backlight results were taken. The S. *epidermis* is slightly higher at 50ppm, which contradicted the 168hr viable count and may be erroneous. The 1.25% sample has the lowest amount of both live and dead bacteria. This was also expected, due to the rapid effect of the 1.25% coat on the bacteria, giving little time for bacterial growth before the MIC was reached. The 50ppm coat had living bacteria with fluorescence slightly over the 1.25% value but had zero CFUs on the plates, showing a bacterial static affect on at least some of the bacteria in the 168hr samples.

The results illustrated in figures 2 to 4 confirm that the antibiotic can be mixed with the sol and used as an effective silica-glass coat without losing much, if any, of its antibiotic activity. The results also confirm that the antibiotic is released over time.

The high temperature results confirm that the gentamicin was still active and released having been dried at 80°C. The 48hr 50ppm high temp coat showed the lowest CFU number of any of the other 48hr 50ppm results, possibly the result of a faster release rate but due to the low number of results there is little evidence of this. However, from the results it was confirmed that high temperature curing of the coat does not have any notable negative affect on gentamicin release or antibiotic activity.

## Claims

1. A substrate having a hybrid coating chemically bonded to the substrate, the coating obtainable by a sol-gel process using a polysiloxane;
the coating comprising a polysiloxane based network of silicon-carbon bonds and silicon-oxygen bonds; and
an antimicrobial component releasably captured within the network and capable of defusing from the network in response to a fluid introduced into the coating.

2. The substrate as claimed in claim 1 wherein the coating is formed as a porous network allowing fluid to flow into and out of the network.

3. The substrate as claimed in claim 1 wherein the network further comprises additional species chemically bonded to the polysiloxane, the additional species within the network derived from any one or a combination of the following:
• a silane;
• a silicate;
• nano particles;
• γ-Al₂O₃;
• TiO₂.

4. The substrate as claimed in claim 1 wherein the network further comprises any one or a combination of the following set of:
• an oxysilane;
• an epoxy siloxane;
• an acrylic siloxane;
• an organically modified silane.

5. The substrate as claimed in claim 1 wherein the polysiloxane based network comprises a substantially linear polysiloxane backbone.

6. The substrate as claimed in claim 5 wherein the polysiloxane comprises any one or a combination of the following organic groups chemically bonded directly to at least one silicone atom of the Si-O backbone:
• an alkyl;
• an aryl;
• a mixed alkyl-aryl.

7. The substrate as claimed in claim 6 wherein the polysiloxane comprises alkyl groups directly bonded to at least one silicone atom of the Si-O backbone, wherein the alkyl group comprises between 1 to 20 carbon atoms.

8. The substrate as claimed in claims 6 wherein the alkyl, aryl and/or mixed alkyl-aryl groups are functionalised by comprising nitrogen, phosphorus, oxygen, sulphur and/or chlorine atoms.

9. The substrate as claimed in claim 6 comprising alkyl, aryl or alkyl-aryl mixed groups directly bonded to at least one silicone atom of the Si-O backbone in addition to at least one functionalised side chain directly bonded to either the Si-O backbone or at least one of the alkyl, aryl or alkyl-aryl side groups;
the functionalised side chain comprising any one or a combination of the following set of:
• an acrylic;
• an epoxy;
• an organosilane.

10. The substrate as claimed in any preceding claim wherein the coating comprises a multilayer structure, the layers comprising different concentrations of silicon-carbon bonds and silicon-oxygen bonds.

11. The substrate as claimed in any preceding claim wherein the coating comprises a multilayer structure, each layer having a different concentration of the antimicrobial.

12. A biocompatible implant for a human or animal, the implant comprising the coating obtainable by a sol-gel process using a polysiloxane according to any preceding claim.

13. A method of preparing a substrate, the method comprising:
preparing a sol using a polysiloxane ;
making a preparation comprising an antimicrobial;
mixing the sol and the antimicrobial component together to form a mixture;
coating the substrate with the mixture;
curing the mixture on the substrate to form a sol-gel derived hybrid coating chemically bonded to the substrate, the coating comprising a polysiloxane based network of silicon-carbon bonds and silicon-oxygen bonds;
wherein the antimicrobial is releasably captured within the network and capable of defusing from the network in response to a fluid introduced onto the coating.

14. The method as claimed in claim 13 wherein the antimicrobial is substantially uniformly distributed through the coating thickness from an external facing region to the substrate-coating interface.

15. The method as claimed in claim 13 wherein the antimicrobial is substantially non-uniformly distributed within a multi-layer system through the coating thickness from an external facing region to the substrate-coating interface.

## Patentansprüche

1. Substrat, das eine Hybridbeschichtung hat, die chemisch an das Substrat gebunden ist, wobei die Beschichtung durch ein Sol-Gel-Verfahren unter Verwendung eines Polysiloxan erhältlich ist;
wobei die Beschichtung ein Polysiloxan-basiertes Netzwerk aus Silicium-Kohlenstoff-Bindungen und Silicium-Sauerstoff-Bindungen umfasst und
wobei eine antimikrobielle Komponente freisetzbar in dem Netzwerk gefangen ist und fähig ist, als Reaktion auf ein in die Beschichtung eingeführtes Fluid aus dem Netzwerk zu entweichen.

2. Substrat, wie es in Anspruch 1 beansprucht ist, wobei die Beschichtung als ein poröses Netzwerk ausgebildet ist, das Fluid in das und aus dem Netzwerk strömen lässt.

3. Substrat, wie es in Anspruch 1 beansprucht ist, wobei das Netzwerk außerdem zusätzliche Spezies chemisch an das Polysiloxan gebunden umfasst, wobei die zusätzliche Spezies in dem Netzwerk von einem beliebigen oder einer Kombination der folgenden abgeleitet ist:
• einem Silan;
• einem Silicat;
• Nanopartikeln;
• γ-Al₂O₃;
• TiO₂.

4. Substrat, wie es in Anspruch 1 beansprucht ist, wobei das Netzwerk außerdem ein beliebiges oder eine Kombination aus der folgenden Gruppe umfasst:
• ein Oxysilan;
• ein Epoxysiloxan;
• ein Acrylsiloxan;
• ein organisch modifiziertes Silan.

5. Substrat, wie es in Anspruch 1 beansprucht ist, wobei das Polysiloxan-basierte Netzwerk eine im Wesentlichen lineare Polysiloxan-Hauptkette umfasst.

6. Substrat, wie es in Anspruch 5 beansprucht ist, wobei das Polysiloxan eine beliebige oder eine Kombination der folgenden organischen Gruppen direkt chemisch gebunden an wenigstens ein Siliciumatom der Si-O-Hauptkette umfasst:
• ein Alkyl;
• ein Aryl;
• ein gemischtes Alkylaryl.

7. Substrat, wie es in Anspruch 6 beansprucht ist, wobei das Polysiloxan Alkylgruppen umfasst, die direkt an wenigstens ein Siliciumatom der Si-O-Hauptkette gebunden sind, wobei die Alkylgruppe zwischen 1 und 20 Kohlenstoffatome umfasst.

8. Substrat, wie es in Anspruch 6 beansprucht ist, wobei die Alkyl-, Aryl- und/oder gemischten Alkylarylgruppen funktionalisiert sind, indem sie Stickstoff-, Phosphor-, Sauerstoff-, Schwefel- und/oder Chloratome umfassen.

9. Substrat, wie es in Anspruch 6 beansprucht ist, das Alkyl-, Aryl- oder gemischte Alkylarylgruppen, die direkt an wenigstens ein Siliciumatom der Si-O-Hauptkette gebunden sind, zusätzlich zu wenigstens einer funktionalisierten Seitenkette, die direkt an entweder die Si-O-Hauptkette oder wenigstens eine der Alkyl-, Aryl- oder Alkylarylseitengruppen gebunden ist, umfasst;
wobei die funktionalisierte Seitenkette eines oder eine Kombination der folgenden Gruppe umfasst:
• ein Acryl;
• ein Epoxy;
• ein Organosilan.

10. Substrat, wie es in einem vorangehenden Anspruch beansprucht ist, wobei die Beschichtung eine Mehrschichtstruktur umfasst, wobei die Schichten unterschiedliche Konzentrationen an Silicium-Kohlenstoff-Bindungen und Silicium-Sauerstoff-Bindungen umfassen.

11. Substrat, wie es in einem vorangehenden Anspruch beansprucht ist, wobei die Beschichtung eine Mehrschichtstruktur umfasst, wobei jede Schicht eine unterschiedliche Konzentration des antimikrobiellen Mittels hat.

12. Biokompatibles Implantat für einen Menschen oder ein Tier, wobei das Implantat die Beschichtung, erhältlich durch ein Sol-Gel-Verfahren unter Verwendung eines Polysiloxans, gemäß einem vorangehenden Anspruch umfasst.

13. Verfahren zur Herstellung eines Substrates, wobei das Verfahren umfasst:
Herstellen eines Sols unter Verwendung eines Polysiloxan;
Herstellen einer Zubereitung, die ein antimikrobielles Mittel umfasst;
Mischen des Sol und der antimikrobiellen Komponente miteinander unter Bildung eines Gemisches;
Beschichten des Substrates mit dem Gemisch;
Härten des Gemisches auf dem Substrat unter Bildung einer von Sol-Gel-stammenden Hybridbeschichtung, die chemisch an das Substrat gebunden ist, wobei die Beschichtung ein Polysiloxan-basiertes Netzwerk aus Silicium-Kohlenstoff-Bindungen und Silicium-Sauerstoff-Bindungen umfasst;
wobei das antimikrobielle Mittel freisetzbar in dem Netzwerk eingefangen ist und fähig ist, als Reaktion auf ein in die Beschichtung eingeführtes Fluid aus dem Netzwerk zu entweichen.

14. Verfahren, wie es in Anspruch 13 beansprucht ist, wobei das antimikrobielle Mittel im Wesentlichen gleichmäßig durch die Beschichtungsdicke von einer äußeren Frontregion zu der Substrat-Beschichtungs-Grenzfläche verteilt ist.

15. Verfahren, wie es in Anspruch 13 beansprucht ist, wobei das antimikrobielle Mittel im Wesentlichen nicht gleichmäßig innerhalb eines Mehrschichtsystems durch die Beschichtungsdicke ab einer äußeren Frontregion zu der Substrat-Beschichtungs-Grenzfläche verteilt ist.

## Revendications

1. Substrat ayant un revêtement hybride chimiquement lié au substrat, le revêtement pouvant être obtenu par un procédé sol/gel utilisant un poly(siloxane),
le revêtement comprenant un réseau à base de poly(siloxane) de liaisons silicium-carbone et de liaisons silicium-oxygène ; et
un composant antimicrobien capturé de façon libérable à l'intérieur du réseau et pouvant se désamorcer du réseau en réponse à l'introduction d'un fluide dans le revêtement.

2. Substrat selon la revendication 1, dans lequel le revêtement est formé comme un réseau poreux permettant à un fluide de s'écouler dans et hors du réseau.

3. Substrat selon la revendication 1, dans lequel le réseau comprend en outre des espèces supplémentaires chimiquement liées au poly(siloxane), les espèces supplémentaires à l'intérieur du réseau étant dérivées de l'un quelconque ou d'une combinaison des éléments suivants :
- un silane ;
- un silicate ;
- des nanoparticules ;
- γ -Al₂O₃;
- TiO₂.

4. Substrat selon la revendication 1, dans lequel le réseau comprend en outre l'un quelconque ou une combinaison des éléments de l'ensemble suivant :
- un oxysilane ;
- un époxy siloxane ;
- un siloxane acrylique ;
- un silane organiquement modifié.

5. Substrat selon la revendication 1, dans lequel le réseau à base de poly(siloxane) comprend un squelette poly(siloxane) sensiblement linéaire.

6. Substrat selon la revendication 5, dans lequel le poly(siloxane) comprend l'un quelconque ou une combinaison des groupes organiques suivants chimiquement liés directement à au moins un atome de silicium du squelette Si-O :
- un alkyle ;
- un aryle ;
- un alkyl-aryle mixte.

7. Substrat selon la revendication 6, dans lequel le poly(siloxane) comprend des groupes alkyle directement liés à au moins un atome de silicium du squelette Si-O, dans lequel le groupe alkyle comprend entre 1 et 20 atomes de carbone.

8. Substrat selon la revendication 6, dans lequel les groupes alkyle, aryle et/ou alkyl-aryle mixte sont fonctionnalisés par le fait de comprendre des atomes d'azote, de phosphore, d'oxygène, de soufre et/ou de chlore.

9. Substrat selon la revendication 6, comprenant des groupes alkyle, aryle ou alkyl-aryle mixte directement liés à au moins un atome de silicium du squelette Si-O en plus d'au moins une chaîne latérale fonctionnalisée directement liée soit au squelette Si-O soit à au moins l'un des groupes latéraux alkyle, aryle ou alkyl-aryle ;
la chaîne latérale fonctionnalisée comprenant l'un quelconque ou une combinaison des éléments de l'ensemble suivant :
- un acrylique ;
- un époxy ;
- un organosilane.

10. Substrat selon l'une quelconque des revendications précédentes, dans lequel le revêtement comprend une structure multicouche, les couches comprenant différentes concentrations de liaisons silicium-carbone et de liaisons silicium-oxygène.

11. Substrat selon l'une quelconque des revendications précédentes, dans lequel le revêtement comprend une structure multicouche, chaque couche ayant une concentration différente de l'antimicrobien.

12. Implant biocompatible pour un être humain ou un animal, l'implant comprenant le revêtement pouvant être obtenu par un procédé sol/gel utilisant un poly(siloxane) selon l'une quelconque des revendications précédentes.

13. Procédé de préparation d'un substrat, le procédé comprenant :
la préparation d'un sol en utilisant un poly(siloxane) ;
la réalisation d'une préparation comprenant un antimicrobien ;
le mélange du sol et du composant antimicrobien ensemble pour former un mélange ;
le revêtement du substrat avec le mélange ;
le durcissement du mélange sur le substrat pour former un revêtement hybride dérivé de sol/gel chimiquement lié au substrat, le revêtement comprenant un réseau à base de poly(siloxane) de liaisons silicium-carbone et de liaisons silicium-oxygène ;
dans lequel l'antimicrobien est capturé de façon libérale à l'intérieur du réseau et peut se désamorcer du réseau en réponse à l'introduction d'un fluide sur le revêtement.

14. Procédé selon la revendication 13, dans lequel l'antimicrobien est distribué de façon sensiblement uniforme à travers l'épaisseur du revêtement depuis une région en regard vers l'extérieur vers l'interface substrat-revêtement.

15. Procédé selon la revendication 13, dans lequel l'antimicrobien est distribué de façon sensiblement non uniforme à l'intérieur d'un système multicouche à travers l'épaisseur du revêtement depuis une région en regard vers l'extérieur vers l'interface substrat-revêtement.
